# EUROPEAN PATENT APPLICATION

(11) **EP 2 401 985 A1**
(43) Date of publication of application: **04.01.2012**
(21) Application number: 10168228.4
(22) Date of filing: 02.07.2010
(51) Int. Cl.: A61F 2/00, A61M 1/10, A61M 1/12

(54) **Medical device comprising an artificial contractile structure**

(71) Applicant: MyoPowers Medical Technologies SA, 1005 Lausanne (CH)
(72) Inventor: Tozzi, Piergiorgio, 1005, Lausanne (CH); Hayoz, Daniel, 1752, Villars-sur-Glâne (CH); Horst, Martin, 6048, Horw (CH); Wieland, Marco, 4054, Bâle (CH); Burkhard, Merg, 64404, Bickenbach (DE); Zeyher, Peter, 64289, Darmstadt (DE)
(74) Representative: GLN

(57) **Abstract**

The present invention relates to a medical device comprising an artificial contractile structure which may be advantageously used to assist the functioning of a hollow organ.

Said medical device comprises an artificial contractile structure comprising at least one contractile element (100) adapted to contract an organ, in such way that said contractile element (100) is in a resting or in an activated position, at least one actuator designed to activate said contractile structure, and at least one source of energy for powering said actuator. Said actuator comprises actuating means designed in such a way that the energy consumption of said medical device is between 50 mAh/year and 2 000 mAh/year for a pressure applied by the contractile element on the organ comprised between 0.1 N/cm² and 5 N/cm².

## Description

### Technical Field

The present invention relates to a medical device comprising an artificial contractile structure activated by an actuator, which may be advantageously used to assist the functioning of an organ, e.g. a sphincter or the heart. More generally, it can be used for moving or constricting a hollow or a tubular part of the body in such a way as to reduce its diameter.

### Background of the invention

It is known to use artificial structures to assist muscular contraction. Such structures are adapted to assist atrial or ventricular contraction, or to assist or replace a natural sphincter. The use of such artificial sphincters has increased in recent years because faecal and urinary incontinences now affects more than 10% of people over 60 years of age and dramatically increases in patients over 80 years of age. Several pharmaceutical or surgical solutions have been developed for treating urinary and faecal incontinences. Generally, the outcome of surgery for treatment of urinary and faecal incontinence has to be regarded as low. The impacts on health care costs and overall quality of life of the patient are enormous.

The AMS800 artificial sphincter for urinary incontinence is commercialized by American Medical Systems and is composed of three components, a cuff, a pump, and a pressure-regulating balloon. The cuff is implanted at the bulbous urethra in males and is inflatable by means of a fluid. The pump is implanted in the scrotum and the pressure-regulating balloon is implanted in the abdomen. The major problems when using AMS800 is the tissue erosion around the urethra due to the constant pressure, the atrophy and irritation of tissues at the location of the inflatable cuff, and the emergency surgery for repair should the device remain in closed position in the event of mechanical failure. All other commercialized artificial sphincters whether for urinary or faecal incontinences bear similar drawbacks.

The ProAct™ artificial sphincter for urinary incontinence is commercialized by Uromedica and is composed of two small implantable balloons. During a short outpatient procedure, the balloons are surgically placed under the skin in the area where the prostate of the patient was surgically treated. The balloons help protect against accidental leaking of urine by increasing the amount of pressure required to urinate. When the patient needs to urinate, a normal amount of effort still should be required to push the urine out. However, the pressure from the balloons will help guard against unintentional urine loss, such as during a sneeze or cough. The major problems when using ProACT™ are identical to the problems using AMS800 artificial sphincter described above.

Flowsecure™, manufactured by Sterilin Ltd, another silicone hydraulic urinary sphincter similar to AMS800, has an extra pressure transmission balloon to transfer increased intra abdominal pressure directly to the cuff. implantation of this device is technically feasible, but still difficult and is reported to be safe and effective in the short-term for the treatment of male urodynamic stress urinary incontinence, arising from a number of etiologies. However, the major problems when using FlowSecure™ are identical to the problems using AMS800 artificial sphincter described above.

Some publications describe the use of artificial sphincters comprising shape memory alloy elements suitable for opening and closing a part of an organ in a living body. EP 1 238 638 describes an artificial sphincter having an opening/closing portion for opening and closing, wherein said opening/closing portion has:
- a pair of elongated shape memory alloy elements that change reversibly between opposite shapes upon changes in temperature, and
- hinges that link said pair of shape memory alloy elements together in a cylindrical shape.

Such artificial sphincter is placed around the intestine of a human or animal inside the body near to an intestinal opening so that the opening/closing portion constricts the intestine. When the shape memory alloy elements are heated, they change shape, so that the constricting force on the intestine is lost.

However, as the opening/closing portion is still constricting the same region of the intestine, there is likely damage to this part of the body, and more especially a risk of tissue erosion, atrophy and burns, due to the constant pressure and heating of the shape memory alloy elements.

Reversible thermal lesions occur when the local temperature is increased to the 42°C to 44°C range (5 C - 7°C over the normal body temperature of 37°C) and that irreversible thermal lesions occur when the local temperature is increased above 45°C (>8°C temperature rise over 37°C, which is the normal temperature). The time over overheating also plays an important role.

Moreover, in normal state, the shape memory alloy elements are not heated and are each bent to constrict the intestine. That means that heating is necessary to open the artificial sphincter. If the heating means fail, the sphincter remains closed and cannot be opened what may be leading to life threatening complications. An emergency surgery is then necessary to open the artificial sphincter to solve the problem.

Another artificial sphincter has been proposed in JP 07-051304. This document describes a constrictor comprising two shape memory alloy elements with different shape memories, and covered by covering materials. The first covering material is formed in a shape to close the urethra in the daytime, and the second covering material is formed in a shape to half close the urethra in the night. This sphincter allows changing the pressure to the urethra, in order to prevent the incontinence in life action in the daytime, and to avoid necrosis of the tissue by loosing the pressure to the urethra in the night.

However, the drawbacks of such artificial sphincters are that there is a risk of tissue erosion and consequential necrosis, due to the high constant pressure to the urethra during the day and that there is a risk of incontinence during the night. If the shape memory alloy is no more efficient or is broken, the whole sphincter should be moved and replaced.

Moreover, JP 07-051304 discloses an artificial sphincter in which the shape memory alloy elements are disconnected from each other. This embodiment does not allow optimal pressure control.

Moreover, this kind of shape memory alloy elements uses a lot of power. That means that the battery needs to be changed very often or alternatively very large batteries have to be used.

EP 1 598 030 discloses a urine incontinence treatment apparatus, comprising a restriction device for engaging the urethra to form a restricted urine passageway in the urethra, the restriction device being operable to change the restriction of the urine passageway, a source of energy, and a control device operable from outside the patient's body for controlling the source of energy to release energy for use in connection with the operation of the restriction device, a motor or pump implantable in the patient, wherein the source of energy is adapted to power the motor or pump and the control device is adapted to control the motor or pump to operate the restriction device. The source of energy can be an internal battery with a lifetime of at least 10 years. However, as disclosed in EP 1 598 030, an internal battery is an advantageous solution for embodiments of the apparatus that have a relatively high consumption of energy, which cannot be satisfied by direct supply of wireless energy. Therefore, even if the lifetime of the internal battery is of 10 years, the operation time of said internal battery is shorter as the energy consumption is very high. Said internal battery should therefore be changed very often.

Therefore there are, at the present time, no adequate solutions, whether commercial or in the literature, for implanting devices comprising an artificial contractile structure, particularly for the treatment of faecal or urinary incontinence.

### Summary of the invention

The present invention provides a medical device comprising an artificial contractile structure which allows to avoid the disadvantages of the prior art.

Accordingly, the present invention relates to a medical device comprising:
- an artificial contractile structure comprising at least one contractile element adapted to contract an organ, in such way that said contractile element is in a resting or in an activated position, the activated position being defined with said contractile element constricting the organ and the resting position being defined with said contractile element not constricting the organ,
- at least one actuator designed to activate said contractile structure,
- at least one source of energy for powering said actuator.

According to the invention, said actuator comprises actuating means designed in such a way that the energy consumption of said medical device is between 50 mAh/year and 2 000 mAh/year for a pressure applied by the contractile element on the organ comprised between 0.1 N/cm² to 5 N/cm².

Advantageously, the source of energy may be selected to have an operation time comprised between 2 months and 10 years, preferably between 1 year and 10 years, and more preferably between 2 years and 8 years, optimally 5 years.

Preferably, the actuator may comprise at least one electromotor and transmission means linked to the contractile element and designed to transmit to the contractile element a force induced by the electromotor.

In some preferred embodiments, the artificial contractile structure may comprise at least two contractile elements being distributed along a support in order to be able to reduce the volume of the organ to be contracted in at least two distinct regions of the organ, and the medical device may comprise at least two actuators respectively linked to their corresponding contractile element by their corresponding transmitting means. The medical device may further comprise a control unit which is adapted to activate each contractile element pulsatory and alternately independently from each other.

In a preferred embodiment, the actuator may be designed so that the contractile element applies, in a pulsating and alternating manner, a pressure on an organ to be contracted during a period comprised between 30 seconds to 60 minutes, preferably between 30 seconds to 45 minutes, and more preferably between 10 minutes and 30 minutes. Preferably, the strength is such that the different regions of the organ are completely closed in a pulsating and alternating manner.

Advantageously, the present invention provides a medical device comprising an artificial contractile structure which is designed for chronic applications (i.e. long-term implantation), for example for many months and preferably many years.

Such devices may be used in several indications, e.g. for assisting or replacing a natural sphincter, especially for the treatment of faecal or urinary incontinence, for assisting atrial or ventricular contraction, for assisting the respiratory function, for assisting or replacing a paralyzed muscle or for treating venous insufficiency. The present invention is particularly designed for improving sphincter muscle function and therefore to improve the patient's quality of life with a significant reduction of treatment costs.

### Brief description of the drawings

Figure 1 is a schematic view of a medical device according to the present invention, the contractile element being in resting position,

Figure 2 is a schematic view of the device of Figure 1, the contractile element being in activated position,

Figure 3 is a schematic view of a control unit used in the invention, and

Figure 4 represents the cycle time as a function of the operating time for a device of the invention using primary batteries.

### Detailed description

In the present description, the term "organ" covers any organ of the human body, preferably an organ comprising a hollow part, containing fluids as for example the ventricular part of the heart, or a region of an organ in the living body having an overall cylindrical shape, for example a blood vessel, the urinary tract, the colon, the stomach or any other body part against which pressure can be applied.

In the present description, the term "electromotor" covers any device designed to produce motion and mechanical effects by the action of electricity.

In the present description, the term "constrict" means that the contractile element applies a pressure against a region of an organ around or on which said contractile element has been placed.

In the present description, the term "pulsatory" means that each contractile element is activated and deactivated in alternation with another contractile element to constrict or apply a pressure or not against the region of the organ or the hollow part around or on which it has been placed, preferably so as to close or open said region of the organ or of the hollow part. More especially, in a preferred embodiment, contractile element one is closed for a certain time, while the other contractile element(s) are open. After a given time the contractile element two will be closed while the contractile element one is still closed. When contractile element two is closed, contractile element one opens, and so on. The frequency of alternate activation is dependent upon the nature of the tissues and inside organ pressure, and is adjusted so that no tissue erosion and burn appear after several months of implantation.

In the present description, the term "link" means a direct or indirect connection between two elements.

According to the invention, the actuator of the medical device comprises:
- an artificial contractile structure comprising at least one contractile element adapted to contract an organ, in such way that said contractile element is in a resting or in an activated position, the activated position being defined with said contractile element constricting the organ and the resting position being defined with said contractile element not constricting the organ,
- at least one actuator designed to activate said contractile structure,
- at least one source of energy for powering said actuator,
- at least one control unit for controlling the actuator.

According to the invention, said actuator comprises actuating means designed in such a way that the energy consumption of said Medical device is between 50 mAh/year and 2 000 mAh/year for a pressure applied by the contractile element on the organ comprised between 0.1 N/cm² and 5 N/cm².

Preferably, said actuating means are designed in such a way that the energy consumption of the medical device is comprised between 70 mAh/year and 1 500 mAh/year for a pressure applied by the contractile element on the organ comprised between 0.3 N/cm² and 2.5 N/cm².

The actuator comprises at least one electromotor linked to the transmission means, which are designed to transmit to the contractile elements a force induced by said electromotor. In a preferred embodiment, said electromotor may comprise an electric motor, a gearhead connected to said motor, a lead screw cooperating with said gearhead, and a nut mounted on said lead screw and linked to said transmissions means. The actuator may further comprise sensors designed to indicate the position of the nut.

The transmission means may be mechanical, hydraulic, electromechanical or pneumatic. Preferably, the transmission means may be a cable linking the nut to the contractile element. The cable may be protected by a coaxial sheath. The sheath can be made for example of silicone, polyimide, PTFE composites (PTFE and fluoroethylkene polymers), pure PTFE, or other appropriate polymers. The sheath can be additionally coated with silicone, if necessary. Cables are well known in surgery. The cables can be made for example out of polyamide like Nylon®, polyether block amide, PTFE, or other appropriate polymers. Alternatively, other materials, as stainless steel or titanium, can be used. Surgeon is used to place cables in the human body. One end of the cable may be connected liquid tight to the contractile element and the other end of the cable is linked liquid tight to the nut of the actuator. Moreover, in some embodiments, one end of the cable may be reversibly connected to the contractile element and the other end of the cable may be reversibly linked to the nut of the actuator in such a way that the cable may be separated from the contractile element or from the actuator.

The source of energy can be implantable or placed outside the body of the patient.

In a preferred embodiment, the actuator and its control unit, and the source of energy are implantable and are placed in the same closed box. In other embodiments, the control unit and the source of energy can be also separated in two boxes (control unit and power supply unit) and connected with an electric cable, which should be easily detachable. In other embodiments, the actuator and its control unit is implantable and the source of energy is placed outside the body of the patient. In some embodiments, the source of energy comprises an implantable rechargeable battery with an implantable antenna and an external battery. Such implantable battery is for example a Lithium-Ion or Lithium Polymer rechargeable battery commercialized by GreatBatch and others. The energy transfer system that is needed to recharge the battery, is preferably through wireless connection. Such system can comprise a recharge unit, as a belt, comprising an external battery. The patient should wear the recharge unit for a number of hours to recharge the implanted battery. The energy should be transmitted wireless to the implanted battery via appropriate antenna. The system can also comprise a cradle for charging the recharge unit. Charging can be performed through a wired or metal contact connection. The battery provides sufficient energy for at least 1 month operation of the medical device. Recharge time is less than 6 hours. In another preferred embodiment, the source of energy is an implantable primary (i.e. non-rechargeable) battery, having a lifetime of at least 4 years.

The features of the battery depend on the application of the artificial structure, on the pressure to be applied, on the number of contractile elements to activate, and how often the patient opens and closes the contractile structure.

In the present invention, when energy is provided to the electromotor, this energy may be transmitted directly to the lead screw which converts its rotative movement to a lateral movement of the nut. When the nut moves along the lead screw, it pulls or pushes the cable to close or open the contractile element. No or minimal energy is needed to maintain the contractile element in its activated position, which means that the maximum pressure on or around the organ is maintained with no or minimal energy consumption.

Energy is only needed to move the nut and close or open the contractile element which also provides significant reduction of the power consumption, that allows a significant increase in the battery life time.

With such lower energy consumption, which was never disclosed in the prior part, the operation time of the battery used as source of energy is comprised between 1 month and 10 years, preferably between 1 year and 10 years, and more preferably between 2 years and 8 years, optimally 5 years.

The medical device of the invention allows therefore the use of a primary battery placed inside the body of the patient, which is to be changed only several years after its implantation, optimally 5 years.

Moreover, the motor, the gear ratio and the lead screw have been chosen in such a way that the travel time needed by the nut for moving along the lead screw between the resting position and the activated position is comprised between 0,2 s and 90 s, for a travel of the nut comprised between 2 mm and 50 mm, preferably between 3 mm and 15 mm. Preferably, the travel time needed by the nut for moving between the resting position and the activated position is comprised between 0,4 s and 60 s, more preferably between 0,5 s and 10 s, and more preferably between 0,5 s and 5 s for a travel of the nut comprised between 2 mm and 50 mm, preferably between 3 mm and 15 mm.

The time for opening or closing the contractile element could be different and depends on the material of the contractile element.

The appropriate electromotor is commercialized for example by Maxon Motor AG, Faulhaber or Portescap. Preferably, the gear ratio is comprised between 4 and 64, and preferably between 4 and 16. The lead screw has a pitch comprised between 1 and 3 and an effective diameter comprised between 2 mm and 4 mm.

The medical device of the invention can comprise only one actuator, the transmission means being designed to transmit the forces induced by the actuator to each of the contractile elements of the structure. In other embodiments, the Medical device can comprise several actuators, each actuator being associated, via appropriate transmission means, to one or several contractile elements.

The artificial contractile structure may be a structure comprising separate contractile elements described above or linked by a support.

In some embodiments, the artificial contractile structure may comprise at least two contractile elements being distributed along a support in order to be able to reduce the volume of the organ to be contracted in at least two distinct regions of said organ, and the device may comprise at least two actuators respectively linked to their corresponding contractile element by their corresponding transmitting means.

If the structure comprises several contractile elements linked by a support, said contractile elements can be designed in such a way that each contractile element is connected to an adjacent contractile element, while remaining flexible one with respect to the other. That means that a contractile element and its adjacent contractile element are physically linked by an appropriate connecting element, allowing one to obtain a compromise between the stiffness and the flexibility of the structure. This structure allow applying to minimal pressure to the tissues avoiding tissue necrosis and damage. Moreover, this structure allows optimal pressure control and implantation of the structure by surgeons, by having a single-piece device which is adaptive to the natural flexibility of the urethra while remaining semirigid so that the structure stays in place and the pressure of each contractile element can be optimally synchronized.

The artificial contractile structure may further comprise a flexible connecting element designed to link each contractile element to an adjacent contractile element, said connecting element being made out of elastic biocompatible material for keeping said contractile elements in longitudinal position while allowing a rotational movement of each contractile element one with respect to the other.

Advantageously, each contractile element is flexible so that it has the freedom to move longitudinally no more than 5 mm to each direction, preferably no more than 3 mm to each direction, and more preferably no more than 1 mm to each direction from an adjacent contractile element, and so that it can move according to a transversal rotation no more than 30°, to each side, preferably no more than 20° to each side from an adjacent contractile element, allowing the most flexibility and independence of each contractile elements from its adjacent contractile elements preventing a peristaltic movement of the whole device along the urethra and allowing optimal synchronization of the contractile elements.

In some embodiments, the control unit may be adapted to pulsatory and alternately activate each contractile element, independently from each other. The actuators are preferably controlled by the same control unit.

In some embodiments, the medical device may further be combined with a device that signals the patient that the contractile structure will open soon, e.g. within next five minutes. This embodiment is preferred if the organ is the bladder, so that the patient has time enough to go to the toilet. The signaling device can be for example a vibration alarm or a LED.

In the invention, the contractile structure is placed around an organ to be contracted or is placed on (or close to) an organ so that a local pressure is applied to such organ. It may comprise one or more contractile elements disposed around the organ.

A medical device of the invention that has one or more contractile elements placed on an organ (so that a local pressure on such organ is achieved, preferably in a pulsatory manner) may be easier to implant for surgeons, because delicate and/or lengthy surgery around the organ is avoided. In the field of incontinence, this device may however be less convenient for full control of incontinence compared to a device whereby the contractile structure is around the urethra. Such medical device (that has one or more contractile elements on an organ) is however superior to the commercial slings used to control urinary incontinence which have poor success rates (see Retropubic versus Transobturator Midurethral Slings for Stress Incontinence, Holly E. Richter et al. The New England Journal of Medecine, 2010; 362:2066-79). Therefore the contractile structure of the medical device of the invention may be designed as a classical sling in terms of shape and dimensions so that a controlled (by the patient) local pressure is applied on the urethra, therefore maximizing control of incontinence. Hereby such device is defined as an "active sling".

This active sling may not be limited by the embodiments of the present invention, meaning that contractile element may be activated mechanically by hydraulic or pneumatic means as described for in the prior art AMS800 device. Preferably, however a source of energy for powering is used, but the energy consumption of said medical device may be even lower than 50 mAh/year for a pressure applied by the contractile element on the organ comprised between 0.1 N/cm² and 5 N/cm². Interestingly even a small pressure on the urethra that is managed by an active sling will improve control of incontinence compared to traditional slings.

Preferably, this active sling is adapted to be placed, at least partially, in a female or male patient in one of several locations, i.e., below the pubis bone, so as to lift the urethra from a point below the pubis bone when the patient is standing, into the pubis bone, so as to lift the urethra from a point attached to the pubis bone of the patient, or above the pubis bone of the patient, so as to lift the urethra from a point above the pubis bone when the patient is standing.

The urethra is lifted by reducing the length of the u-shaped traditional sling. Normally the device forms a loop and the adjustment changes the length of the loop to lift the urethra. The loop can have any shape or form that can be used to lift the urethra when placed inside the loop, when implanted. The device forms a loop that is placed around stable tissue. The loop holds up the urethra, when placed inside the loop, when implanted. Preferably, the interconnecting part is a band or a thread, or a plurality of bands or threads connected to each other to lift the urethra.

The resting position of the contractile element of the structure corresponds to a state in which any force is transmitted by the transmitting means to the contractile element, and the activated position corresponds to a state in which a force has been transmitted in such a way that the contractile element closes and constricts the organ to be contracted.

In some embodiments, the contractile element is made out of biocompatible materials, preferably selected from the group consisting of silicone and polytetrafluorethylene (PTFE), polylactide (PLA)-polymer, polyurethane (PUR), Polymethylmethacrylate (PMMA), polyoxymethylene (POM), HDPE polyethylene and LDPE polyethylene or combinations thereof. Other appropriate material as other polymers or metal can be used.

The contractile element of the contractile structure may have the form of an open ring to be placed around the organ or around a hollow part of the organ to be contracted, said ring having a moving part linked to the transmitting means.

Preferably, the contractile element comprises a moving part linked to the actuator and designed to move, when activated by the actuator, between the activated position and the resting position of the contractile element.

Advantageously, the contractile element comprises a band which surrounds at least partially the organ to be contracted, and the transmission means are designed to be linked to one end of the band and to pull it, when the contractile element is activated by the actuator, in such a way that said contractile element reaches its activated position.

Preferably, the transmitting means are a cable, and the band may comprise at one end a point for linking the cable and at the other end a hole crossed by said cable.

In some embodiments, the size of the band may be comprised between 4 cm and 15 cm in length, preferably between 4 cm and 10 cm in length, and between 3 mm and 15 mm in width, preferably between 3 mm and 12 mm in width.

The control unit and/or power supply unit includes electronics and software designed to:
- control and adjust the actuator generating the force transmitted to the contractile element
- provide control of the actuator from outside the body through wireless connection
- optionally recharge the internal battery through wireless connection
- control the status of the battery
- provide test and diagnosis support for health care professionals
- handling of alarm conditions and exceptions.

The control unit comprises a microprocessor that distributes current to actuators so that they activate the contractile elements pulsatory, at the required pressure and at the required frequency.

The microprocessor can be adjusted via remote control individually for each patient regarding pressure and frequency of opening and closing.

Ideally these adjustments can be done after implantation transcutaneously, preferably by a medicinal physician in order to optimize control of volume reduction (such as incontinence leaking), Readjustments can be performed at any time during the life time of the device using a remote control, as described below.

The number of contractile elements to contract can be adapted to the required pressure to apply on the organ. For example, in the case of urinary sphincter, the number of contractile elements to open and close can be adapted to the abdominal pressure.

The pressure of the structure on the region of the organ to be contracted may be comprised between 0.1 N/cm² and 5 N/cm², and preferably between 0.3 N/cm² and 2.5 N/cm².

In a preferred embodiment, the device of the invention comprises:
i) an artificial contractile structure implantable into the human body and comprising one or more contractile elements able to be activated by an actuator as described above,
ii) at least one implantable actuator which upon activation will induce a contraction of the contractile elements, such as the actuators described above,
wherein the actuator and the contractile elements are designed so that the pressure, applied on the organ to be contracted, is comprised between 0.1 N/cm² and 5 N/cm², and preferably between 0.3 N/cm² and 2.5 N/cm² during a period comprised between 30 seconds and 60 minutes, preferably between 30 seconds and 45 minutes, and more preferably between 10 minutes and 30 minutes.

Each contractile element is preferably activated or deactivated several times a day, and most preferably several times an hour. The contractile elements may be activated, in a pulsating and alternating manner, a pressure on an organ to be contracted during a period comprised between 30 seconds and 60 minutes, preferably between 30 seconds and 45 minutes, and more preferably between 10 minutes and 30 minutes. The relaxation time is dependent on the number of regions which are to be contracted by the independent contractile elements.

If the artificial structure is adapted to contract for example four regions of an organ, and if only one contractile element is activated at the same time, each contractile element can be activated during one minute and deactivated during three minutes in an alternating manner. In another embodiment, each contractile element can be activated during five minutes and deactivated during fifteen minutes in an alternating manner. If the structure is adapted to contract three regions of an organ, each contractile element can be activated during one minute and deactivated during two minutes in an alternating manner. If the structure is adapted to contract two regions of an organ, it comprises two contractile elements, which can be activated during 30 minutes and deactivated during 30 minutes in an alternating manner.

The activation of each contractile element can be random or sequential.

Only one of the contractile elements or several contractile elements can be contracted at the same time. In other embodiments, one contractile element can remain contracted or closed whereas another contractile element is contracted or closed.

All these embodiments are obtained by means of an adequate control unit. Said control unit is designed to allow an adjustment of the pressure of the contractile structure on the organ according to the patient's need, by adjusting the force generated by the actuator and the frequency the contractile structures are acting. Advantage is that the physician can customize the optimal pressure of the contractile structure to side effects on the organs, for example by means of a magnet placed around the device. The parameters of the control unit and also of the actuator can be adjusted by the physician after the implantation of the device during the postoperative consultations.

The control of the contractile structure and more especially its opening can be achieved, by the physician or the patient himself, by a manual control of the control unit by means of a remote control to open and close the urethra. The remote control is preferably wireless. For the physician, the remote control can be designed to enable adjustments of the medical device (activation force, parameters of the pulsatory and alternately activation, test and diagnosis mode). An optical signal may be provided in order to show the patient the level of the battery status. Two different remote controls can be provided: a simple remote control for the patient and an advanced remote control for the healthcare professionals. The patient gets a simple remote control to open and close the contractile structure and to get few information like battery status, device status... The healthcare professionals have an advanced remote control that in addition allows to readjust the pressure and frequency, move the device into the examination mode as described below (motor will move typically 5mm in the opposite direction of closing the contractile structure).

For emergency, the control unit may be controlled by means of a switch placed under the skin, which is activated by pressure on one or several buttons. Preferably, the switch comprises several buttons and the sequence for pressing the buttons is predetermined in order to avoid accidental opening of the structure.

In other embodiments, the control of the contractile structure and more especially its opening can be achieved, by the physician or the patient himself, by a manual control of the contractile elements themselves by means of a releasing device designed to manually open the contractile structure. Such releasing device can be used if the patient lost the remote control or if a surgeon wishes to open the structure to endoscopically examine the patient or if a kidney stone has to be removed. This corresponds to the examination mode (motor will move typically 5mm in the opposite direction of closing the contractile structure to totally open the contractile structure) allowing the examination with an endoscope without risk of damage of the urethra.

Advantageously, the closed structure of the invention has a diameter comprised between 8 mm and 25 mm. The dimensions of the open structure are such that, when the contractile element(s) of the structure is/are fully open, the surgeon can move an endoscope through the lumen of the urethra/rectum in order to endoscopically examine the patient. In the same way, the dimensions of the open structure are such that, when the contractile elements of the structure are fully open, kidney stone removal is possible.

Preferably, each contractile element is separated from an adjacent contractile element no less than 2 mm to 2 cm, preferably 3 mm to 1 cm, more preferably 4 mm to 8 mm, for avoiding over-compression.

Preferably, the structure of the invention may be comprise between 2 and 8 contractile elements, so that it makes an overall length comprised between 20 mm and 50 mm.

### Examples:

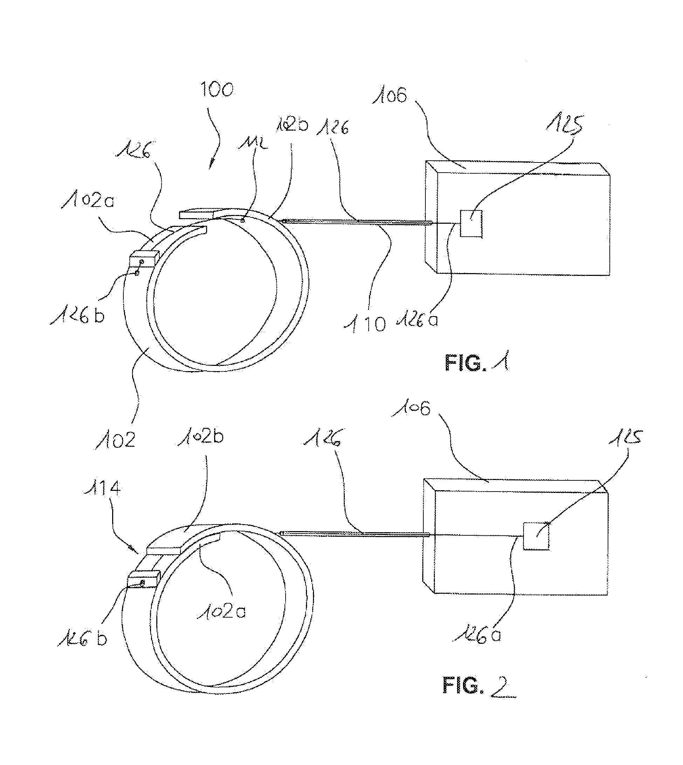

Referring to Figures 1 and 2, one embodiment of the medical device of the invention, used to treat urinary incontinence, comprises a contractile element 100 designed to surround partially a hollow part of the urethra, for example. For simplification of the drawings, only one contractile element 100 is shown. But the medical device of the invention may comprise a contractile structure comprising at least two contractile elements 100 adapted to be placed around the hollow part of the urethra, for example, and linked by connecting elements.

The contractile element 100 comprises a band 102 designed to surround at least one time the hollow part of the organ to be contracted. The band 102 is made of silicone, PTFE, PLA, PUR, PMMA, (POM), HDPE LDPE or combination thereof to reduce the friction when the band wraps closely around the organ. Other appropriate material, such as metal, can be used.

The medical device comprises also an actuator placed in a box 106 away from the organ to be contracted. Such an actuator is linked to the contractile element 100 by a cable 126.

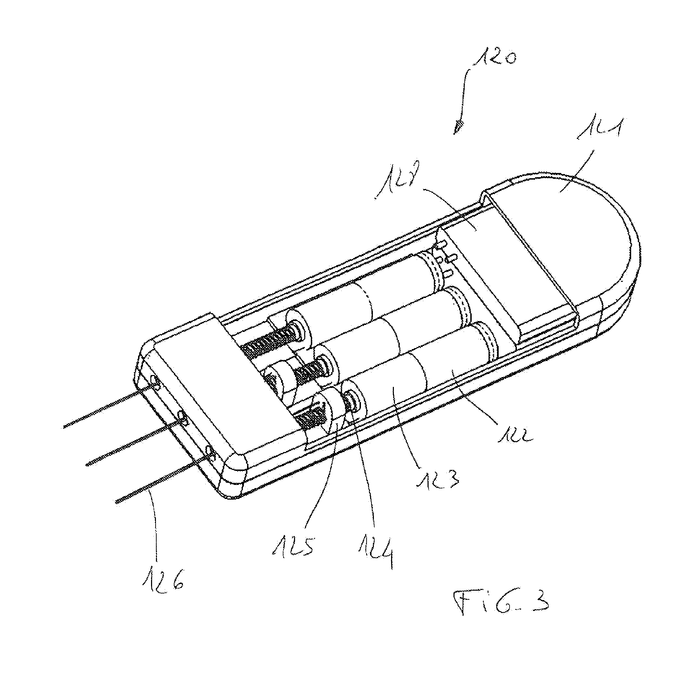

Figure 3 shows a control unit 120 used to control and activate the contractile element 100 shown in Figures 1 and 2. The control unit 120 is placed in a box 121 made of polymer or titanium. The control unit 120 comprises three actuators, each having an electromotor comprising an electric motor 122, a gearhead 123 connected to said motor 122, a lead screw 124 cooperating with said gearhead 123, and a nut 125 mounted on said lead screw 124. The nut 125 is connected to the cable 126 that transmits the force to the corresponding contractile element 100 to close or open it. The cable 126 is made of stainless steel, titanium or polymer and surrounded by a coaxial sheath 110 of silicon. One end 126a of the cable 126 is connected liquid tight and may be reversibly linked to the nut 125. The other end 126b of the cable 126 is linked liquid tight and may be reversibly linked to one end 102a of the band 102. The other end 102b of the band 102 comprises a hole 112 through which the cable 126 runs.

Soft foam could be placed in the space 114 between the band 102 and the cable 126 to avoid tissue in-growth between the cable 126 and the contractile element 100. Alternatively, the sliding surfaces of the band could be modified to prevent tissue in-growth, for example by coating.

Each nut 125 moves along the corresponding lead screw 124 to close or open the corresponding contractile element 100.

The control unit 120 comprises also a printed circuit board to control the actuators and batteries 128, for example rechargeable batteries. A percutaneous energy transfer supply can be developed for battery recharge.

A travel sensor is provided in such a way that the control unit 120 knows the exact position of the nuts 125 and therefore the position of each contractile element 100. It is also needed for the readjustment of the force.

In figure 1, the contractile element 100 has not been contracted. The nut 125 is closer to the contractile element 100, which is in a resting position, the band 102 being loosely wrapped around the organ.

When an electric current is applied to an electromotor by the control unit 120, the corresponding lead screw 124 rotates in such a way that the corresponding nut 125 is moving along the corresponding lead screw 124. If the nut 125 moves away from the contractile element 100, the nut 125 pulls on the corresponding cable 126, which pulls on the corresponding contractile element 100 to close it. More especially, the nut 125, by moving away from the contractile element 100, moves the end 126a of the cable 126 into the box 121. So that the other end 126b of the cable 126 is moved as the same way. By moving, the end 126b of the cable 126 pulls on the end 102a of the band 102 which slides under the other end 102b, until the band 102 is closely wrapped around the organ to constrict it. The contractile element 100 is then in an activated position as shown by Figure 2.

No energy is needed to maintain the contractile element 100 in its activated position.

When the contractile element 100 has to come back in its resting position, the control unit 120 supplies electrical energy to the electromotor, in such a way that the lead screw 124 rotates in the opposite direction. The nut 125 comes closer to the contractile element 100. Then, the cable 126 is not pulled by the nut 125 any more in such a way that the contractile element 100 comes back to its resting position as shown by Figures 1.

Several contractile elements 100 can be used to form a contractile structure. In such a case, such structure comprises a support along which the contractile elements are distributed in order to constrict the organ in distinct regions. Each contractile element is linked to its actuator by the corresponding transmitting means. The control unit is therefore adapted to distribute current to each actuator, preferably in order to pulsatory and alternately contract the contractile elements 100.

In this case, there are several gates which can be independently, pulsatory and alternately activated in order to contract one or the other region around which the contractile elements 100 have been placed, in a pulsating and alternating manner. This allows an alternate contraction along the urethra, several times an hour. Such a configuration avoids stressing of the underlying tissue followed by erosion and necrosis.

The control unit is designed to activate at least one actuator and therefore to activate at least one contractile element so that at least one region of the urethra is closed to avoid incontinence. The patient deactivates the device if necessary, so that each actuator is inactivated to open each region of the hollow part of the urethra, allowing the passage of the urine.

There are also means for opening on demand said artificial contractile structure, used by the physician or the patient himself to inactivate the actuators and open the contractile elements.

The device can further comprise sensing means selected from pressure, and force sensing means.

Obviously, the device of the invention can be used with a control unit adapted to drive the contraction of the contractile elements, on demand, without pulsatory and alternately contracting said contractile elements.

The operating time of the medical device as shown by Figure 3 was tested for different travels of the nut 125 and for different cycle times. The travel is the distance covered by the nut 125 moving along the lead screw 124 in such a way that the contractile element 100 moves between its resting and activated positions. A cycle time comprises movement of the nut for closing the contractile element, time for which the contractile element is closed, movement of the nut for opening the contractile element and time for which the contractile element is opened.

The travels were 10 mm, 8 mm and 5 mm. The cycle times were 10 minutes, 20 minutes and 30 minutes.

The electromotor comprised the motor 08GS61 from Portescap, lead screw pitch is 1.80 mm and the diameter is 2.00 mm; gear ratio is 16.

The control unit comprises as source of energy two primary batteries of 1.1 Ah, with an assumed shelf life of 1 year.

The pressure applied by the contractile element on the organ was 1,5 N.

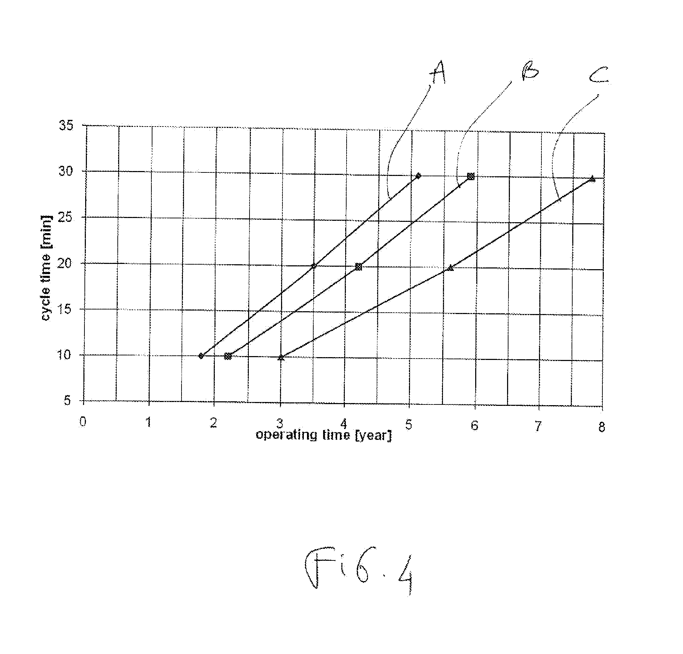

The results are shown by Figure 4, which represents the cycle time as a function of the operating time for different travels for a travel of the nut of 10 mm (curve A), a travel of 8 mm (curve B) and a travel of 5 mm (curve C). Figure 4 shows that the medical device of the invention allows to use primary batteries enabling to obtain an operating time of 1,8 years to 7,8 years.

This medical device comprising primary batteries was compared with a similar medical device but using a rechargeable battery of 200 mAh.

The travel of the nut was 10 mm and the pressure applied by the contractile element on the organ was 1,5 N.

In the first case, the cycle time was 10 minutes and in the second case, the cycle time was 30 minutes.

The results are shown in the Table below:

| **Type of power supply** | **Volume power supply** | **Typical operating time before exchange** / **recharge** | |
|---|---|---|---|
| | | cycle time **= 10min.** | cycle time **= 30min.** |
| Rechargeable Battery | 3.3ml + TET | 2 months | 5.5 months |
| 200 mAh | | | |
| Primary Battery | 7.4ml | 1.8 years +1 year shelf life | > 5 years +1 year shelf life |
| 2x1.1 Ah | | | |

The Table shows that the medical device of the invention using an electromotor and a primary battery has an operating time of more than 5 years before exchange of the battery, with a cycle time of 30 minutes, and of 2 years with a cycle time of 10 minutes.

Moreover, such a medical device allows applying minimal pressure to the tissues thereby avoiding tissue necrosis and damage, even if each contractile element applies a pressure at a frequency of 30 to 45 minutes alternately with the other contractile elements. That means that every contractile element is closed for 30 to 45 minutes alternately with the other contractile elements. A device as AMS 800 shows erosion because the device is closed for about 6 to 8 hours per night and during the day for about 4 hours, assuming that the patient goes every 4h to the toilet.

## Claims

1. Medical device comprising:
- an artificial contractile structure comprising at least one contractile element (100) adapted to contract an organ, in such way that said contractile element (100) is in a resting or in an activated position, the activated position being defined with said contractile element (100) constricting the organ and the resting position being defined with said contractile element (100) not constricting the organ,
- at least one actuator designed to activate said contractile structure,
- at least one source of energy for powering said actuator,
**characterized in that** said actuator comprises actuating means designed in such a way that the energy consumption of said medical device is between 50 mAh/year and 2 000 mAh/year for a pressure applied by the contractile element on the organ comprised between 0.1 N/cm² and 5 N/cm².

2. Medical device according to claim 1, **characterized in that** said actuating means are designed in such a way that the energy consumption of the medical device is comprised between 70 mAh/year and 1 500 mAh/year for a pressure applied by the contractile element on the organ comprised between 0.3 N/cm² and 2.5 N/cm².

3. Medical device according to anyone of claims 1 and 2, **characterized in that** the source of energy is selected to have an operation time comprised between 2 months and 10 years, preferably between 1 year and 10 years, and more preferably between 2 years and 8 years.

4. The medical device according to anyone of claims 1 to 3, **characterized in that** said actuator further comprises at least one electromotor.

5. The medical device according to claim 4, **characterized in that** said actuator further comprises transmission means (126) linked to the contractile element (100) and designed to transmit to the contractile element (100) a force induced by the electromotor.

6. The medical device according to claim 4 and 5, **characterized in that** said electromotor comprises an electric motor (122), a gearhead (123) connected to said motor (122), a lead screw (124) cooperating with said gearhead (123), and a nut (125) mounted on said lead screw (124) and linked to said transmission means (126).

7. The medical device according to anyone of claims 5 to 6, **characterized in that** said transmission means (126) are mechanical, hydraulic, electromechanical or pneumatic.

8. The medical device according to claims 6 and 7, **characterized in that** the transmission means (126) are a cable linking the nut (125) to the contractile element (100).

9. The medical device according to claim 6, **characterized in that** the actuator further comprises sensors designed to indicate the position of the nut (125).

10. The medical device according to anyone of claims 1 to 9, **characterized in that** the contractile element (100) comprises a moving part linked to the actuator and designed to move, when activated by the actuator, between the activated position and the resting position of the contractile element (100).

11. The medical device according to anyone of claims 5 to 10, **characterized in that** the contractile element (100) comprises a band (102) which surrounds at least partially the organ to be contracted, and **in that** the transmission means (126) are designed to be linked to one end of the band and to pull it, when the contractile element is activated by the actuator, in such a way that said contractile element (100) reaches its activated position.

12. The medical device according to anyone of claims 1 to 11, **characterized in that** the source of energy comprises an implantable rechargeable battery with an implantable antenna and an external battery.

13. The medical device according to anyone of claims 1 to 11, **characterized in that** the source of energy is an implantable primary battery.

14. The medical device according to anyone of claims 1 to 13, **characterized in that** the artificial contractile structure comprises at least two contractile elements being distributed along a support in order to be able to reduce the volume of the organ to be contracted in at least two distinct regions of the organ, and **in that** the medical device comprises at least two actuators respectively linked to their corresponding contractile element by their corresponding transmitting means.

15. The medical device according to claim 14, **characterized in that** each contractile element is connected to an adjacent contractile element, while remaining flexible one with respect to the other.

16. The medical device according to claim 15, **characterized in that** the artificial contractile structure further comprises a flexible connecting element designed to link each contractile element to an adjacent contractile element, said connecting element being made out of elastic biocompatible material for keeping said contractile elements in longitudinal position while allowing a rotational movement of each contractile element one with respect to the other.

17. The medical device according to anyone of claims 14 to 16, **characterized in that** it further comprises a control unit which is adapted to activate each contractile element pulsatory and alternately independently from each other.

18. The medical device according to anyone of claim 1 to 17, **characterized in that** the actuator is designed so that the contractile element applies a pressure on an organ to be contracted during a period comprised between 30 seconds and 60 minutes, preferably between 30 seconds and 45 minutes, and more preferably between 10 minutes and 30 minutes.

19. Use of the medical device according to anyone of claims 1 to 18 for assisting or replacing a natural sphincter.

20. Use of the medical device according to anyone of claims 1 to 18 for assisting or replacing a paralyzed muscle.
